# EUROPÄISCHE PATENTSCHRIFT

(11) **EP 1 569 715 B1**
(45) Veröffentlichungstag und Bekanntmachung des Hinweises auf die Patenterteilung: **12.12.2012**
(21) Anmeldenummer: 03773720.2
(22) Anmeldetag: 28.11.2003
(51) Int. Cl.: A61N 1/365, A61N 1/362

(54) **BIVENTRIKULÄRER HERZSCHRITTMACHER ZUR KARDIALEN RESYNCHRONISATIONSTHERAPIE**
BIVENTRICULAR CARDIAC PACEMAKER FOR CARDIAC RESYNCHRONISATION THERAPY
STIMULATEUR CARDIAQUE BIVENTRICULAIRE POUR THERAPIE DE SYNCHRONISATION CARDIAQUE

(30) Priorität: 02.12.2002 DE 10257156
(43) Veröffentlichungstag der Anmeldung: 07.09.2005
(73) Patentinhaber: BIOTRONIK SE & Co. KG, 12359 Berlin (DE)
(72) Erfinder: Militello, Carmelo, 00041 Albano Laziale (IT)
(74) Vertreter: Lindner-Vogt, Karin L.
(86) Internationale Anmeldenummer: PCT/EP2003/013450
(87) Internationale Veröffentlichungsnummer: WO 2004/050177

(56) Entgegenhaltungen:
- EP-A- 0 793 976
- WO-A-00/78391
- WO-A-99/30777
- WO-A-03/051457
- US-A- 5 782 884
- US-A- 5 902 325
- US-A- 6 134 472
- US-A1- 2001 012 953

## Beschreibung

Die Erfindung betrifft einen Herzschrittmacher mit einem Stimulationsimpulsgenerator für eine biventrikuläre Stimulation eines Herzens. Der Stimulationsimpulsgenerator ist mit rechtsventrikulären Elektroden für die Stimulation eines rechten Ventrikels des Herzens und mit linksventrikulären Elektroden für die Stimulation eines linken Ventrikels des Herzens zu verbinden. Statt eines Stimulationsimpulsgenerators können auch zwei separate Stimulationsimpulsgeneratoren für den rechten und linken Ventrikel vorhanden sein. Der Herzschrittmacher weist außerdem eine Steuereinrichtung beispielsweise in Form einer Steuereinheit auf, die mit dem Stimulationsimpulsgenerator oder den ventrikulären Stimulationsimpulsgeneratoren verbunden und ausgebildet ist, biventrikuläre Stimulationsmodi und insbesondere eine durch die Steuereinrichtung einstellbare, interventrikuläre Verzögerungszeit, mit der rechts- und linksventrikuläre Stimulationsimpulse zeitverzögert auszulösen sind, zu steuern.

Derartige Schrittmacher für die biventrikuläre Stimulation sind grundsätzlich bekannt und werden insbesondere für eine kardiale Resynchronisationstherapie (CRT = Cardiac Resynchronisation Therapy) eingesetzt.

Herzschrittmacher dienen in der Regel dazu, insbesondere ein menschliches Herz mit Hilfe elektrischer Stimulationsimpulse zu Kontraktionen anzuregen, wenn solche Kontraktionen auf natürliche Weise nicht oder nicht zum rechten Zeitpunkt eintreten.

Bekannt ist es, Zeitpunkte oder Zeitfenster festzulegen, in denen ein solcher Herzschrittmacher eine natürliche Kontraktion erwartet. Wenn eine derartige natürliche Kontraktion auftritt, wird ein künstlicher Stimulationsimpuls in der Regel unterdrückt (inhibiert), der ansonsten zum möglichst geeigneten Zeitpunkt abgegeben wird. Es ist ebenfalls bekannt, unter bestimmten Umständen und abhängig vom Betriebsmodus des Herzschrittmachers einen Stimulationsimpuls auf jeden Fall abzugeben, unabhängig davon, ob eine natürliche Kontraktion erfasst wird oder nicht. Die damit verbundenen Schrittmacherbetriebsarten werden international einheitlich durch den dritten Buchstaben eines Dreibuchstaben-Codes wie "VVI" oder "DDD" gekennzeichnet.

Geeignete Zeitpunkte können auf vielfältige Art und Weise ermittelt werden. Beispielsweise können die physiologisch geeigneten Zeitpunkte für eine Kontraktion des rechten Ventrikels eines Herzen durch Abfühlen (Sensing) des rechten Atriums eines Herzens ermittelt werden. Die natürliche oder die stimulierte Kontraktion des rechten Ventrikels erfolgt nach einer atrio-ventrikulären Verzögerungszeit, die so bemessen ist, dass sich eine möglichst optimale Pumpleistung durch die zeitlich aufeinanderfolgende Kontraktion von Atrium und Ventrikel ergibt.

Darüber hinaus ist es auch bekannt, falls erforderlich das Atrium zu stimulieren.

Die durch den Schrittmacher vorgegebene Herzrate kann in allen Fällen künstlich aus einem für den physiologischen Bedarf eines Patienten charakteristischen Messwert abgeleitet werden, falls sie nicht im Falle einer atrium-synchronen Stimulation durch einen natürlichen und gesunden atrialen Rhythmus vorgegeben ist. Der physiologische Bedarf richtet sich dabei nach einem Anstrengungs- oder Erregungszustand des Patienten. Eine physiologisch adäquate Bestimmung einer Stimulationsrate kann bei einem derartigen ratenadaptiven Schrittmacher auch dadurch erfolgen, dass die intrakardiale Impedanz für jeweils einen Herzzyklus bestimmt wird und eine geeignete Stimulationsrate aus diesem Impedanzwert abgeleitet wird.

Die angedeuteten Betriebsarten betreffen in der Regel Schrittmacher, die entweder ausgebildet sind, Erregungspotential im rechten Ventrikel aufzunehmen und Stimulationsimpulse an einen rechten Ventrikel abzugeben oder zusätzlich auch Erregungspotential im rechten Atrium aufzunehmen und gegebenenfalls Stimulationsimpulse an das rechte Atrium abzugeben. Die damit verbundenen Schrittmacherbetriebsarten werden international einheitlich mit dem Dreibuchstaben-Code wie beispielsweise DDD oder VVI bezeichnet.

In jüngerer Zeit wird neben dem rechten Atrium und dem rechten Ventrikel auch der linke Ventrikel stimuliert. Dies erfolgt häufig gleichzeitig mit dem rechten Ventrikel, kann aber auch mit einer gewissen Verzögerung gegenüber dem linken Ventrikel erfolgen. Eine damit verbundenen Herzschrittmachertherapie des Herzens wird als Cardiac Resynchronisation Therapy (CRT) bezeichnet.

Ein biventrikulärer Schrittmacher, bei dem die interventrikuläre Verzögerungszeit einstellbar ist und der eine gemessene Bioimpedanz des Patienten auswertet, ist aus US 2001/0012953 bekannt.

Ein Herzschrittmachertherapie gemäß der Präambel des Anspruches 1 ist aus WO 99/30777 bekannt.

Die Erfindung bezieht sich insbesondere auf den Aspekt der optimalen bi-ventrikulären Stimulation, die alle vorgenannten Aspekte eines Schrittmachers ergänzen kann. Der Erfindung liegt insbesondere die Aufgabe zu Grunde, einen Schrittmacher für eine optimierte kardiale Resynchronisationstherapie anzugeben.

Diese Aufgenbe wird durch die in Anspruch 1 angegebene Merkmale geläst.

Erfindungsgemäß wird diese Aufgabe dadurch gelöst, dass die Steuereinheit des Schrittmachers der Eingangs genannten Art mit einer Impedanzerfassungseinheit verbunden ist, die mit intrakardialen Elektroden zum Zwecke der Impedanzmessung zu verbinden ist. Die Impedanzerfassungseinheit ist ausgebildet, aus einem von der Impedanzerfassungseinheit gebildeten, von der intrakardialen Impedanz abhängigen Eingangssignal ein einen optimalen biventrikulären Stimulationsmodus anzuzeigendes Ausgangssignal zu bilden, und zwar indem die Steuereinheit (30) ausgebildet ist, diejenige interventrikuläre Verzögerungszeit einzustellen bei der die zweite Ableitung des Verlaufs der intracardialen Impedanz während eines Herzzyklusses am größten ist.

In einer bevorzugten Ausführungsvariante ist die Steuereinheit dazu ausgebildet, ein die interventrikuläre Verzögerungszeit bestimmendes Ausgangssignal zu bilden. Neben der interventrikulären Verzögerungszeit ist ein jeweiliger biventrikulärer Stimulationsmodus auch dadurch bestimmt, ob überhaupt beide Ventrikel stimuliert werden oder nur einer von beiden. In diesem Sinne ist die Steuereinheit dazu ausgebildet für einen grundsätzlich biventrikulären Herzschrittmacher zu ermitteln, ob es tatsächlich optimal ist, beide Ventrikel zu stimulieren, und falls ja, mit welcher Verzögerungszeit. Falls die Auswertung der intrakardialen Impedanz durch die Steuereinheit ergeben sollte, dass nur einer der beiden Ventrikel zu stimulieren ist, wird auch der optimale Ventrikel ermittelt.

Zur Bestimmung des optimalen biventrikulären Stimulationsmodus ist die Steuereinheit vorzugsweise dazu ausgebildet, verschiedene biventrikuläre Stimulationsmodi auszulösen und für jeden Stimulationsmodus die intrakardiale Impedanz auszuwerten. Die verschiedenen Stimulationsmodi unterscheiden sich dadurch, ob keiner oder nur jeweils einer von beiden Ventrikeln (unechte biventrikuläre Stimulation, hier auch als univentrikuläre Stimulation bezeichnet) oder beide Ventrikel (echte biventrikuläre Stimulation) stimuliert werden. Im Falle der Stimulation beider Ventrikel unterscheiden sich verschiedene Submodi des echten biventrikulären Stimulationsmodus durch voneinander verschiedene interventrikuläre Verzögerungszeiten.

Die Erfindung beruht auf der Erkenntnis, dass aus der intrakardialen Impedanz, und zwar insbesondere aus deren zweiter Ableitung, abzulesen ist, wie gut der linke und der rechte Ventrikel eines Herzens bei einer biventrikulären Herzschrittmachertherapie vor allem im Rahmen einer Resynchronisationstherapie miteinander synchronisiert sind. Aus dieser neuen Erkenntnis leitet sich die Lehre ab, Messwerte für die intrakardiale Impedanz insbesondere zum Zwecke der Optimierung der interventrikulären Verzögerungszeit auszuwerten. Dies erlaubt es, nicht nur das Ermitteln sondern auch das Einstellen der Parameter einer optimalen biventrikulären Stimulation innerhalb eines Herzschrittmachers zu automatisieren, in dem eine Steuereinheit intrakardial erfasste Impedanzwerte auswertet und aus diesen ein Steuersignal ableitet, welches die Stimulationsparameter, insbesondre die interventrikuläre Überleitungszeit bestimmt.

Alternativ zu einer Herzschrittmacher-internen, automatischen Bestimmung des optimalen biventrikulären Stimulationsmodus ist es auch möglich und vorgesehen, dass die Steuereinheit aus den Messwerten für die intrakardiale Impedanz für verschiedene interventrikuläre Verzögerungszeiten ein Ausgangssignal generiert, welches die physiologisch günstigste der verschiedenen interventrikulären Verzögerungszeiten anzeigt. Dieses Ausgangssignal wird vorzugsweise telemetrisch auf ein externes Gerät übertragen und jeweils zusammen mit dem dazugehörigen biventrikulären Stimulationsmodus, insbesondre der interventrikulären Verzögerungszeit einem Arzt angezeigt. Der Arzt kann dann ebenfalls auf telemetrischen Wege die interventrikuläre Verzögerungszeit anhand der angezeigten Ausgangsignale optimal einstellen.

Vorzugsweise lässt sich die interventrikuläre Verzögerungszeit zwischen 20 und 40 ms einstellen.

Zusätzlich ist die Steuereinheit dazu ausgebildet, mit verschiedenen Elektrodenkonfigurationen oder Elektrodenpositionen korrespondierende Ausgangssignale aus den Messwerten für die intrakardial Impedanz abzuleiten. Diese Ausgangssignale werden vorzugsweise ebenfalls telemetrisch auf ein extrakorporales Gerät übertragen und dort angezeigt. Dies ermöglicht es einem Arzt beispielsweise bereits beim Implantieren der rechtsventrikulären und/oder der linksventrikulären Elektrode diese optimal unter Berücksichtigung der intrakardial erfassten Impedanzwerte zu positionieren.

Im Falle von Elektrodenleitungen mit einer Mehrzahl von Elektroden, die dem ventrikulären Stimulationsgenerator zugeordnet werden können, ist es möglich, die optimal wirksame Elektrodenkonfiguration mit Hilfe der gemessenen Impedanzwerte zu bestimmen. Dies kann wie zuvor beschrieben durch einen Arzt mittels externer Programmierung der optimalen Elektrodenkonfiguration auf telemetrischem Wege erfolgen. Dazu wird das aus den intrakardialen Impedanzwerten von der Steuereinheit ermittelte Ausgangssignal auf telemetrischem Wege auf ein extra-korporales Gerät übertragen und dort angezeigt. Die Bestimmung der optimalen Elektrodenkonfiguration kann jedoch auch automatisch innerhalb des Herzschrittmachers erfolgen, indem die Steuereinheit mit einer Auswahleinheit für die in Frage kommenden, ventrikulären Stimulationselektroden verbunden ist und diese Auswahleinheit in Abhängigkeit von den erfassten, intrakardialen Impedanzwerten ansteuert.

In alternativen Varianten ist die Steuereinheit ausgebildet, genau das Ausgangssignal für das Einstellen der optimalen interventrikulären Verzögerungszeit oder alternativ ein Ausgangssignal für das Bestimmen einer optimalen Elektrodenposition oder -konfiguration aus der zweiten Ableitung des intrakardialen Impedanzverlaufs eines Herzzyklusses abzuleiten. In alternativen Varianten, nicht gemäβ des Erfindung, kann die Steuereinheit auch ausgebildet sein, das im zuvor genannten Absatz genannte Ausgangssignal allein vom Maximalwert der Impedanz während eines Herzzyklusses abzuleiten.

Für die intrakardiale Impedanzmessung ist es vorzugsweise vorgesehen, die Impedanz durch Messen einer Spannung zwischen zwei intrakardialen Elektroden zu ermitteln. Diese beiden Elektroden befinden sich vorzugsweise auf verschiedenen Elektrodenleitungen und zwar zum einen dem linken Ventrikel zugeordnet und zum anderen dem rechten Ventrikel zugeordnet. Ein Messstrom, der für die zuvor genannte zu messende Spannung ursächlich ist, wird vorzugsweise zwischen zwei von den Elektroden für die Spannungsmessung unterschiedlichen Elektroden erzeugt. Bevorzugte Elektroden für das Einleiten des vorzugsweise konstanten Messstroms sind zum einen das Schrittmachergehäuse und zum anderen eine weitere intrakardiale Elektrode, die vorzugsweise auf der Elektrodenleitung des rechten Ventrikels angeordnet ist.

Der Strom für die Impedanzmessung hat vorzugsweise eine im wesentlichen konstante Stromstärke zwischen 100 und 500 mA, vorzugsweise 200 mA. Der Strom für die Impedanzmessung wird vorzugsweise in biphasigen Stromimpulsen angegeben. Die Dauer eines Stromimpulses beträgt vorzugsweise zwischen 20 und 40 Mikrosekunden, besonders bevorzugt 30 Mikrosekunden. Die Stromimpulse werden vorzugsweise mit einer Wiederholrate zwischen 100 und 150 Hertz, vorzugsweise 128 Hertz wiederholt. Die Impedanzerfassungseinheit ist vorzugsweise ausgebildet, die Impedanz in einem Zeitfenster von 50 bis 300 ms Dauer zu ermitteln. Dieses Zeitfenster wird vorzugsweise mit Erfassen eines linksventrikulierenden Ereignisses gestartet. Ein linksventrikuläres Ereignis ist beispielsweise eine natürliche Kontraktion des linken Ventrikels oder auch ein überschwelliger Stimulationsimpuls an den linken Ventrikel.

Vorzugsweise ist die Impedanzerfassungseinheit ausgebildet, einen gemittelten Impedanzverlauf über mehrere Herzzyklen zu erfassen. Dabei wird der jeweilige Stimulationsmodus über die zu mittelnden Herzzyklen konstant gehalten. Der erfasste intrakardiale Impedanzverlauf wird durch die Impedanzerfassungseinheit vorzugsweise tiefpassgefiltert, und zwar mit einer oberen Grenzfrequenz von vorzugsweise 10 Hz.

Der auf diese Weise durch Mittelung und Tiefpassfilterung gewonnene intrakardiale Impedanzverlauf wird durch die Steuereinheit ausgewertet, indem von diesem Impedanzverlauf die zweite Ableitung gebildet wird. Unter der Annahme, dass der intrakardiale Impedanzverlauf jeweils das Blutvolumen in einem Herzen wiederspiegelt, ist aus dem Maximum der zweiten Ableitung dieses intrakardialen Impedanzverlaufs die maximale Beschleunigung abzulesen, die das Blut im Herzen erfährt. Dieser Wert kann mit der Kontraktilität des linken Ventrikels korreliert werden.

Außerdem ist die Impedanzerfassungseinheit oder die Steuereinheit dazu ausgebildet, einen oder mehrere der folgenden Parameter aus dem intrakardialen Impedanzverlauf zu bestimmen:
Z_{ED} als eine Grundlinie für den Impedanzverlauf,
Z_{ES} als die maximale innerhalb eines Herzzyklusses auftretende Impedanz,
T_{ES} als der Zeitabstand zwischen QRS-Komplex (Beginn der Kontraktion des Herzens) und Zeitpunkt, zu dem die maximale interkardiale Impedanz Z_{ES} auftritt,
Zₘᵢₙ als die minimale Impedanz während eines Herzzyklusses,
Tₘᵢₙ als die Zeitdauer, beginnend mit einem QRS-Komplex bis zum Auftreten der minimalen intrakardialen Impedanz Zₘᵢₙ.

Weiterhin ist die Impedanzerfassungseinheit oder die Steuereinheit ausgebildet, aus den zuvor genannten Größen abgeleitete Größen zu ermitteln, nämlich:
die Differenz (Z_{ES} - Z_{ED}) zwischen der maximalen Impedanz Z_{ES} und der Grundlinienimpedanz Z_{ED},
die Differenz (Z_{ES} - Zₘᵢₙ) zwischen der maximal erfassten Impedanz Z_{ES} und der minimal erfassten Impedanz Zₘᵢₙ (Impedanzhub während eines Herzzyklusses) sowie
der Quotient ((Z_{ES} - Zₘᵢₙ) / T_{ES}) aus Impedanzhub (Z_{ES} - Zₘᵢₙ) und der Zeit T_{ES}, sowie
der Quotient ((Z_{ES} - Zₘᵢₙ) / (T_{ES}- Tₘᵢₙ)), gebildet durch den Impedanzhub geteilt (Z_{ES} - Zₘᵢₙ) durch die Differenz (T_{ES}- Tₘᵢₙ) zwischen T_{ES} und Tₘᵢₙ,
die Maxima Z'ₘₐₓ und Z"ₘₐₓ der ersten und der zweiten Ableitung und der intrakardialen Impedanz sowie
die jeweiligen Zeitabstände T'ₘₐₓ und T"ₘₐₓ, beginnend mit einem QRS-Komplex bis zu dem Zeitpunkt, an dem ein Maximum der ersten bzw. der zweiten Ableitung der intrakardialen Impedanz auftritt.

Darüber hinaus ist der Herzschrittmacher vorzugsweise als Zweikammer-Schrittmacher ausgebildet, der atriale und ventrikuläre Stimulationseinheiten umfasst sowie atriale und ventrikuläre Erfassungseinheiten zum Erfassen ventrikulärer bzw. atrialer Ereignisse.

Außerdem ist der Schrittmacher vorzugsweise als ratenadaptiver Schrittmacher ausgebildet, bei dem die Stimulationsrate durch einen für den physiologischen Bedarf eines Patienten charakteristischen Messwert gesteuert wird.

Besonders bevorzugt ist ein ratenadaptiver Herzschrittmacher, bei dem die Stimulationsrate anhand einer Auswertung der intrakardialen Impedanz eingestellt wird. Dieses Konzept ist als Closed-loop stimulation bekannt und stellt auf die Auswertung der intrakardialen Impedanz durch Integralbildung ab.

Weiterhin ist ein Herzschrittmacher bevorzugt, der Mittel zum Erfassen eines Stimulationserfolges (Capture control) aufweist. Diese Mittel sind vorzugsweise dazu ausgebildet, ebenfalls auf den intrakardialen Impedanzverlauf anzusprechen.

Für einen Herzschrittmacher, der die vorgestellte Optimierung des biventrikulären Stimulationsmodus durch Auswertung der intrakardialen Impedanz bietet, ergibt sich für den Fall, dass weiterhin eine Ratenadaption im Sinne der Closed-loop stimulation (Auswertung des Integrals der interkardialen Impedanz) und möglicherweise auch noch eine Impedanz basierte Stimulationserfolgskontrolle (Capture control) aufweist, der Vorteil, dass mit einer Impedanzerfassungseinheit eine Vielzahl der Abläufe in einem Herzschrittmacher steuerbar sind.

Die Erfindung soll nun anhand von Ausführungsbeispielen mit Hilfe der Figuren näher erläutert werden.

Von den Figuren zeigen
- Figur 1a und 1b:: jeweils einen Überblick über ein menschliches Herz mit implantierten Elektrodenleitungen und daran angeschlossenen Herzschrittmacher;
- Figur 2:: ein beispielhaftes, schematisches Blockschaltbild für einen Herzschrittmacher gemäß Figur 1; und
- Figur 3:: ein Diagramm eines intrakardialen Impedanzverlaufs mit Darstellung der aus dem intrakardialen Impedanzverlauf abgeleiteten Parameter.

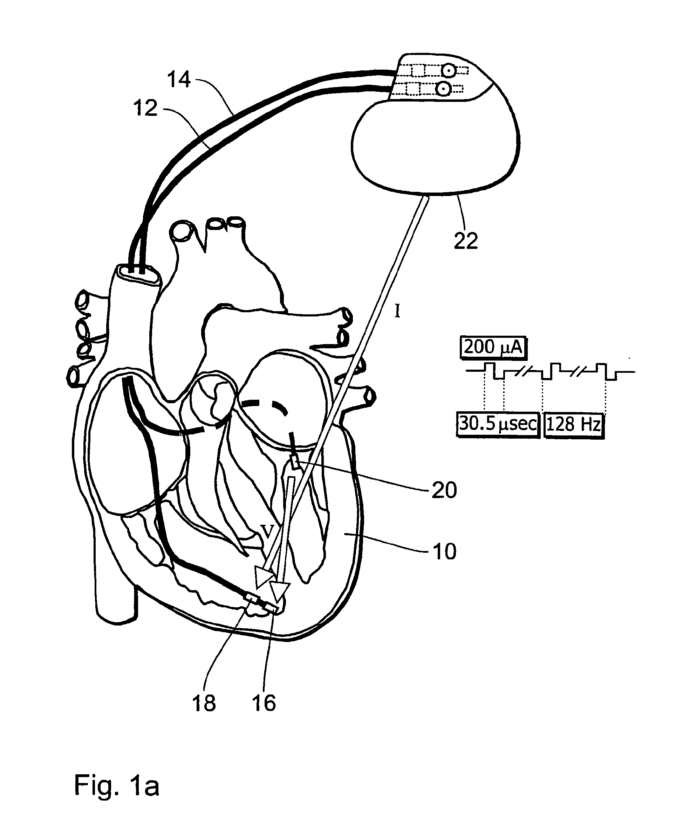

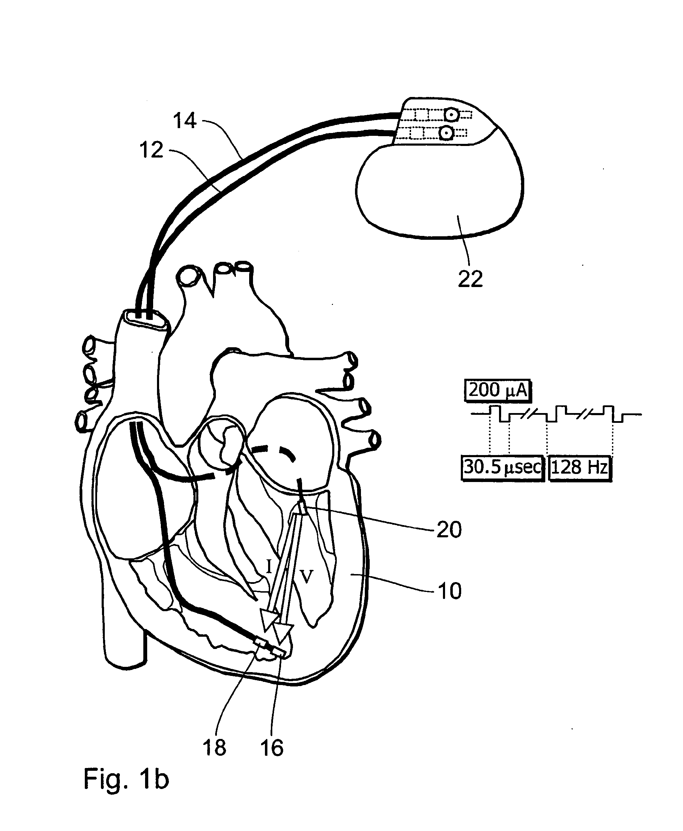

In Figuren 1a und 1b ist ein menschliches Herz 10 abgebildet, in welches intrakardial jeweils eine rechtsventrikuläre Elektrodenleitung 12 und eine linksventrikuläre Elektrodenleitung 14 eingeführt sind. Die rechtsventrikuläre Elektrodenleitung 12 ist am distalen Ende mit zwei Elektroden versehen, nämlich einer Tipelektrode 16 und einer Ringelektrode 18. Die rechtsventrikuläre Elektrodenleitung 12 kann darüber hinaus im Bereich des Atriums auch atriale Stimulations- und Sensingelektroden aufweisen, die im Ausführungsbeispiel nicht dargestellt sind.

Die linksventrikuläre Elektrodenleitung 14 ist über den Coronar Sinus des Herzens 10 und einen von diesem Coronar Sinus abzweigenden lateralen Seitenast eingeführt, so dass eine Spitzenelektrode 20 der linksventrikuläre Elektrodenleitung 14 am linken Ventrikel des Herzens 10 anliegt. Beide Elektrodenleitungen sind mit einem Herzschrittmacher 22 verbunden.

In den Figuren 1a und 1b sind durch Pfeile Strompfade und Spannungsmesspfade eingezeichnet, die diejenigen Elektroden miteinander verbinden, die für die Aufnahme des intrakardialen Impedanzverlaufs zum Zwecke der Bestimmung des optimalen bi-ventrikulären Stimulationsmodus verwendet werden.

In Figur 1a ist eine quadropolare Elektrodenanordnung vorgestellt, bei der Schrittmachergehäuse und die rechtsventrikuläre Ringelektrode 18 dem Einleiten eines zweiphasig gepulsten Stroms für die Impedanzmessung dienen, während eine Spannungsmessung zur Impedanzbestimmung zwischen der linksventrikulären Elektrode 20 und der rechtsventrikulären Tipelektrode 16 stattfindet.

Figur 1b zeigt eine alternative tripolare Anordnung, bei der die Spannungsmessung wie bei der quadropolaren Anordnung aus Figur 1a zwischen der linksventrikulären Elektrode 20 und der rechtsventrikulären Tipelektrode 16 stattfindet. Der zweiphasig gepulste Strom wird jedoch zwischen linksventrikulärer Elektrode 20 - die damit eine Doppelfunktion übernimmt - und der rechtsventrikulären Ringelektrode 18 eingeleitet.

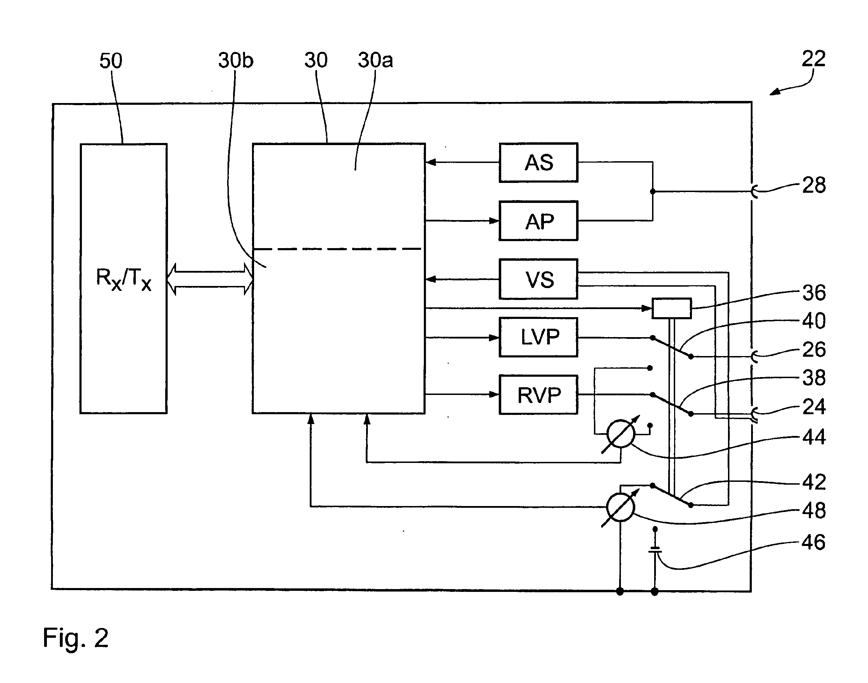

Ein stark vereinfachtes Blockschaltbild des Herzschrittmachers 22 ist in Figur 2 abgebildet. Der Herzschrittmacher 22 ist in üblicher Weise mit Anschlüssen 24 und 26 für die rechts- bzw. die linksventrikuläre Elektrodenleitung 12 und 14 versehen. Der Anschluss 24 ist dabei zweipolig, um die beiden Elektroden 16 und 18 der rechtsventrikulären Elektrodenleitung 12 separat zu kontaktieren. Außerdem ist ein atrialer Elektrodenleitungsanschluß 28 vorgesehen. Der Herzschrittmacher 22 weist eine rechtsventrikuläre Stimulationseinheit RVP, eine linksventrikuläre Stimulationseinheit LVP, eine ventrikuläre Erfassungseinheit VS, eine atriale Stimulationseinheit AP und eine atriale Erfassungseinheit AS auf. Diese Stimulations- bzw. Erfassungseinheiten sind auf die in Figur 2 dargestellte Weise zum einen mit den Elektrodenleitungsanschlüssen 24 bis 28 und zum anderen mit einer Steuereinheit 30 verbunden. Die Steuereinheit 30 umfasst Untereinheiten 30a, die der Ansteuerung der atrialen Stimulationseinheit AP oder der Aufnahme atrialer Ereignisse mittels der atrialen Erfassungseinheit AS dienen.

Mit diesem atrialen Steuerteil 30a verbunden ist eine Untereinheit 30b der Steuereinheit 30, die als ventrikuläres Steuerteil fungiert. Der ventrikuläre Steuerteil 30b ist mit der ventrikulären Erfassungseinheit VS sowie den beiden ventrikulären Stimulationseinheiten LVP und RVP verbunden. Außerdem ist der ventrikuläre Steuerteil mit einer Impedanzerfassungseinheit 34 verbunden, die über einen Wechselschalter 36 mit einem Wechselschaltkontakt 38 mit dem Elektrodenleitungsanschluss 24 und einem weiteren Wechselschaltkontakt 40 mit dem Elektrodenleitungsanschluss 26 zu verbinden. Dies ermöglicht es, die intrakardiale Impedanz über die Tipelektrode der linksventrikulären Elektrodenleitung 14 sowie über die Ringelektrode der rechtsventrikulären Elektrodenleitung 12 zu erfassen. In einer Schaltstellung des Wechselschalters 36 ist die Impedanzerfassungseinheit 34 mit den genannten Elektroden verbunden, in einer anderen Schaltstellung des Wechselschalters 36 sind jeweils die rechte und die linke ventrikuläre Stimulationseinheit RVP und LVP mit den genannten Elektroden verbunden.

Ein dritter Wechselschaltkontakt 42 dient dazu, eine Konstantstromquelle 44 mit der Tipelektrode der rechtsventrikulären Elektrodenleitung 12 zu verbinden. Der andere Pol der Konstantstromquelle 44 ist mit dem Schrittmachergehäuse verbunden. In der nicht dargestellten Schaltstellung des dritten Wechselschaltkontaktes 42 wird zur Impedanzmessung über die Konstantstromquelle 44 ein Strom zwischen Schrittmachergehäuse und Tipelektrode der rechtsventrikulären Elektrodenleitung 12 erzeugt. Dieser Konstantstrom führt zu einem Spannungsabfall, der über die Impedanzerfassungseinheit 34 und die Tipelektrode der linksventrikulären Elektrodenleitung 14 sowie die Ringelektrode der rechtsventrikulären Elektrodenleitung 12 zu messen ist.

Die Konstantstromquelle 44 ist so ausgebildet, dass sie biphasische Strompakete von insgesamt 30,5 µs Dauer erzeugt. Diese biphasischen Stromimpulse werden mit einer Rate von 128 Hz wiederholt. Die Stromstärke ist unterschwellig und beträgt vorzugsweise 200 µA.

Die durch die Impedanzerfassungseinheit 44 aufgenommenen Impedanzwerte werden zu einem intrakardialen Impedanzverlauf zusammengestellt, der mit einer oberen Grenzfrequenz von 10 Hz tiefpassgefiltert wird.

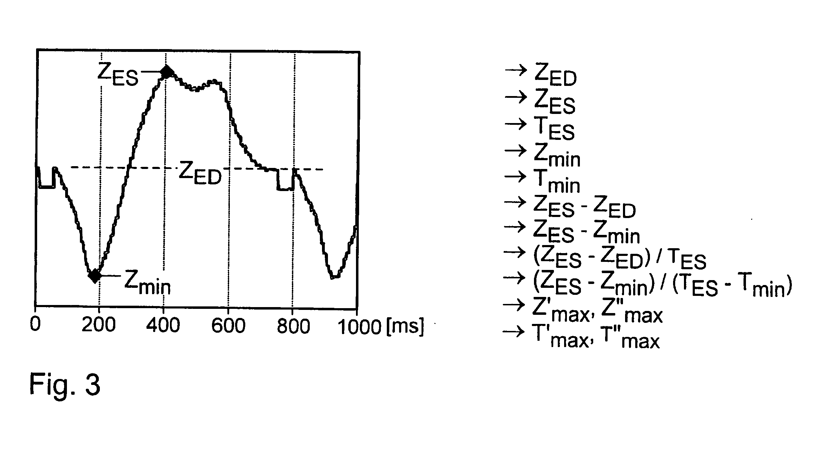

Durch die Steuereinheit 30 gesteuert werden für verschiedene biventrikuläre Stimulationsmodi jeweils intrakardiale Impedanzverläufe über mehrere Herzzyklen (bei jeweils einem Stimulationsmodus) erfasst und gemittelt. Aus diesen gemittelten, tiefpassgefilterten Impedanzverläufen werden die eingangs genannten Parameter Z_{ED}, Z_{ES}, T_{ES}, Zₘᵢₙ, Tₘᵢₙ, Z_{ES} - Z_{ED}, Z_{ES} - Zₘᵢₙ (Z_{ES} - Zₘᵢₙ) / T_{ES}, (Z_{ES} - Zₘᵢₙ) / (T_{ES} - Tₘᵢₙ), Z'ₘₐₓ, Z"ₘₐₓ. T'ₘₐₓ und T"ₘₐₓ gebildet; siehe Figur 3.

Jeweils das Maximum der zweiten Ableitung des intrakardialen Impedanzverlaufes Z"ₘₐₓ wird für jeweils einen biventrikulären Stimulationsmodus gespeichert. Die Steuereinheit 30 bestimmt Z"ₘₐₓ für alle zur Verfügung stehenden biventrikulären Stimulationsmodi. Die Steuereinheit 30 bestimmt das Maximum dieser so ermittelten zweiten Ableitung des intrakardialen Impedanzverlaufes Z"ₘₐₓ und ermittelt auf diese Weise den zugehörigen, biventrikulären Stimulationsmodus als optimal.

Die verschiedenen Stimulationsmodi unterscheiden sich dadurch, dass entweder kein, oder nur der rechte, nur der linke oder beide Ventrikel über die ventrikulären Stimulationseinheiten LVP und RVP stimuliert werden. In dem Fall, in dem beide Ventrikel, also sowohl der rechte als auch der linke Ventrikel des Herzens 10 mittels der beiden ventrikulären Stimulationseinheiten RVP und LVP stimuliert werden, unterscheiden sich die ventrikulären Stimulationsmodi durch die Verzögerungszeit, mit der der linke Ventrikel über die linksventrikuläre Stimulationseinheit LVP nach dem rechten Ventrikel mittels der rechtsventrikulären Stimulationseinheit RVP angesteuert wird. Mit anderen Worten: Zunächst wird der rechte Ventrikel über die rechtsventrikuläre Stimulationseinheit RVP stimuliert, dann verstreicht die interventrikuläre Verzögerungszeit, schließlich wird der linke Ventrikel über die linksventrikuläre Stimulationseinheit LVP stimuliert. Der auf diese Weise ermittelte, optimale biventrikuläre Stimulationsmodus wird durch die Steuereinheit 30 selbsttätig eingestellt.

Alternativ hierzu ist es möglich, die erfassten Werte über eine Telemetrieeinheit 50 auf ein extrakorporales Gerät zu übertragen. Insbesondere werden auf dem extrakorporalen Gerät angezeigt, welcher biventrikulärer Stimulationsmodus gerade vorliegt und welches der dazugehörige Wert Z"ₘₐₓ ist. Ein Arzt kann über das extrakorporale Gerät mehrere biventrikuläre Stimulationsmodi ansteuern und die dazugehörigen Werte für Z"ₘₐₓ bestimmen. Auf diese Weise kann der Arzt den optimalen biventrikulären Stimulationsmodus ermitteln und über den Telemetriesender/Empfänger 50 und die Steuereinheit 30 einstellen.

Es versteht sich, dass der hier beschriebene Herzschrittmacher 22 eine Vielzahl an sich bekannter Funktionen verwirklichen kann und insbesondere in allen bekannten Betriebsmodi, insbesondere DDD (als Zweikammerschrittmacher) und VVI betrieben werden kann.

Der Schrittmacher 22 ist insbesondere dazu ausgebildet, den rechten und den linken Ventrikel über die entsprechenden Stimulationseinheiten RVP und LVP atriumsynchron anzusteuern. Dies bedeutet, das rechts- und linksventrikuläre Stimulationsimpulse nach Ablauf einer atrio-ventrikulären Verzögerungszeit (rechter Ventrikel) bzw. einer atrio-ventrikulären und zusätzlich einer interventrikulären Verzögerungszeit (linker Ventrikel) ausgelöst werden, die mit einem von der atrialen Erfassungseinheit AS erfassten atrialen Ereignis beginnt.

Ein an sich bekanntes Mode-Switching in den WI-Modus bei pathologischen atrialen Raten ist ebenfalls vorgesehen.

Eine Anpassung der Stimulationsrate, mit der der Schrittmacher 22 die Ventrikel und gegebenenfalls das Atrium stimuliert, an den physiologischen Bedarf des Patienten geschieht vorzugsweise im Sinne einer close-loop Stimulation dadurch, dass die Stimulationsrate durch die Steuereinheit 30 von einem aus dem intrakardialen Impedanzverlauf abgeleiteten Integral abhängig eingestellt wird.

Schließlich ist die Steuereinheit 30 auch dazu ausgebildet, einen Stimulationserfolg durch Auswertung des Intrakardialen Impedanzverlaufes zu überwachen und gegebenenfalls die Intensität der durch die Stimulationseinheiten AP, LVB, RVP abgegebenen Stimulationsimpulse anzupassen. Dieses Konzept der Capture-Control mit Anpassung der Stimulationsintensität ist an sich bekannt. Ebenfalls ist die Steuereinheit 30 ausgebildet, für den Fall, dass der intrakardiale Impedanzverlauf keinen Stimulationserfolg anzeigt, einen Sicherheitsimpuls auszulösen.

Um einen Stimulationserfolg bereits während der Abgabe eines ventrikulären Stimulationsimpulses erfassen zu können, ist eine zweite Impedanzerfassungseinheit 48 vorgesehen. Diese ist so geschaltet, dass ein kurzfristiger Impedanzabfall im Myocard bereits während der Stimulationsimpulsabgabe oder kurz darauf zu detektieren und als Indikator für einen Stimulationserfolg auszuwerten.

Im Rahmen von Ergänzungen des vorgestellten Herzschrittmachers kommen insbesondere auch solche Ergänzungen in Frage, den Herzschrittmacher zum Kardioverter/Defibrillator weiterbilden.

## Patentansprüche

1. Herzschrittmacher mit einem Stimulationsimpulsgenerator (RVP; LVP) für eine biventrikuläre Stimulation eines Herzens, welcher mit wenigstens einer rechtsventrikulären Elektrode (16, 18) für die Stimulation eines rechten Ventrikels des Herzens und mit wenigstens einer linksventrikulären Elektrode (20) für die Stimulation eines linken Ventrikels des Herzens zu verbinden und mit einer Steuereinheit (30) verbunden sowie ausgebildet ist, rechts- und linksventrikuläre Stimulationsimpulse mit einer mittels der Steuereineinheit einstellbaren interventrikulären Verzögerungszeit auszulösen, wobei die Steuereinheit (30) mit einer Impedanzerfassungseinheit (34) verbunden ist, die mit intrakardialen Elektroden (16, 18, 20) zu verbinden und ausgebildet ist, aus einem von der Impedanzerfassungseinheit (34) gebildeten, von der intrakardialen Impedanz abhängigen Eingangssignal ein einen optimalen biventrikulären Stimulationsmodus anzeigendes Ausgangssignal zu bilden,
**dadurch gekennzeichnet, dass** die Steuereinheit (30)
ausgebildet ist, diejenige interventrikuläre Verzögerungszeit einzustellen, bei der die zweite Ableitung des Verlaufs der intrakardialen Impedanz während eines Herzzyklusses am größten ist.

2. Herzschrittmacher nach Anspruch 1 , **dadurch gekennzeichnet, dass** die Steuereinheit (30) dazu ausgebildet ist, verschiedene biventrikuläre Stimulationsmodi auszulösen und für jeden Stimulationsmodus die intrakardiale Impedanz auszuwerten.

3. Herzschrittmacher nach Anspruch 1 oder 2, **dadurch gekennzeichnet, dass** die Steuereinheit (30) ausgebildet ist, aus einem von der Impedanzerfassungseinheit (34) gebildeten, von der intrakardialen Impedanz abhängigen Eingangssignal ein eine optimale interventrikuläre Verzögerungszeit anzeigendes Ausgangssignal zu bilden.

4. Herzschrittmacher nach einem der Ansprüche 1 bis 3, **dadurch gekennzeichnet, dass** die Steuereinheit (30) ausgebildet ist, aus einem von der Impedanzerfassungseinheit (34) gebildeten, von der intrakardialen Impedanz abhängigen Eingangssignal ein die interventrikuläre Verzögerungszeit bestimmendes Ausgangssignal zu bilden.

5. Herzschrittmacher nach einem der Ansprüche 1 bis 4, **dadurch gekennzeichnet, dass** interventrikuläre Verzögerungszeit zwischen 20 und 40 ms einstellbar ist.

6. Herzschrittmacher nach einem der Ansprüche 1 bis 5, bei dem der Stimulationsimpulsgenerator (RVP; LVP) mit unterschiedlichen oder hinsichtlich ihrer Position im Herzen veränderbaren ventrikulären Elektroden zu verbinden ist, **dadurch gekennzeichnet, dass** die Steuereinheit (30) ausgebildet ist, intrakardiale Impedanzen für verschiedene Elektrodenkonfigurationen oder Elektrodenpositionen auszuwerten und eine optimale Elektrodenposition oder -konfiguration anzuzeigen.

7. Herzschrittmacher nach einem der Ansprüche 1 bis 6, **dadurch gekennzeichnet, dass** die Impedanzerfassungseinheit (34) ausgebildet ist, die Impedanz über eine Spannungsmessung zu erfassen, die zwischen zwei Elektroden (16, 20) unterschiedlicher Elektrodenleitungen (12, 14) erfolgt.

8. Herzschrittmacher nach einem der Ansprüche 1 bis 7, **dadurch gekennzeichnet, dass** der Herzschrittmacher (22) ausgebildet ist, zur Impedanzmessung einen Strom zwischen einem Schrittmachergehäuse und einer intrakardialen Elektrode zu erzeugen.

9. Herzschrittmacher nach Anspruch 7 und 8, **dadurch gekennzeichnet, dass** die Elektroden für die Spannungsmessung verschieden sind von den Elektroden für das Erzeugen des Stroms für die Impedanzmessung.

10. Herzschrittmacher nach Anspruch 8 oder 9, **dadurch gekennzeichnet, dass** der Herzschrittmacher ausgebildet ist, einen Strom für die Impedanzmessung zu erzeugen, der eine im wesentlichen konstante Stromstärke zwischen 100 und 500µA, vorzugsweise 200 µA hat.

11. Herzschrittmacher nach einem der Ansprüche 8 bis 10, **dadurch gekennzeichnet, dass** der Herzschrittmacher ausgebildet ist, zur Impedanzmessung biphasische Stromimpulse zu erzeugen.

12. Herzschrittmacher nach Anspruch 11, **dadurch gekennzeichnet, dass** der Herzschrittmacher ausgebildet ist, die biphasischen Stromimpulse mit einer Wiederholrate von 100 bis 150 Hz, vorzugsweise 128 Hz zu erzeugen.

13. Herzschrittmacher nach Anspruch 11 oder 12, **dadurch gekennzeichnet, dass** der Herzschrittmacher ausgebildet ist, biphasische Stromimpulse mit einer Impulsdauer zwischen 20 und 40 µs, vorzugsweise etwa 30 µs zu erzeugen

14. Herzschrittmacher nach einem der Ansprüche 1 bis 13, **dadurch gekennzeichnet, dass** die Impedanzerfassungseinheit (34) oder die Steuereinheit (30) ausgebildet ist, die Impedanz in einem Zeitfenster von 50 bis 300 ms Dauer zu mitteln.

15. Herzschrittmacher nach Anspruch 14, **dadurch gekennzeichnet, dass** die Impedanzerfassungseinheit (34) oder die Steuereinheit (30) ausgebildet ist, das Zeitfenster mit Erfassen eines linksventrikulären Ereignisses (Kontraktion) zustarten.

16. Herzschrittmacher nach Anspruch 14, **dadurch gekennzeichnet, dass** die Impedanzerfassungseinheit (34) oder die Steuereinheit (30) ausgebildet ist, einen intrakardialen Impedanzverlauf zu berechnen.

17. Herzschrittmacher nach Anspruch 14, **dadurch gekennzeichnet, dass** die Impedanzerfassungseinheit (34) oder die Steuereinheit (30) ausgebildet ist, einen oder mehrere der folgenden Parameter des Intrakardialen Impedanzverlaufs zu bestimmen:
Z_{ED} als eine Grundlinie für den Impedanzverlauf,
Z_{ES} als die maximale innerhalb eines Herzzyklusses auftretende Impedanz,
T_{ES} als der Zeitabstand zwischen QRS-Komplex (Beginn der Kontraktion des Herzens) und Zeitpunkt, zu dem die maximale interkardiale Impedanz Z_{ES} auftritt,
Zₘᵢₙ als die minimale Impedanz während eines Herzzyklusses,
Tₘᵢₙ als die Zeitdauer, beginnend mit einem QRS-Komplex bis zum Auftreten der minimalen intrakardialen Impedanz Zₘᵢₙ,
die Differenz (Z_{ES}-Z_{ED}) zwischen der maximalen Impedanz Z_{ES} und der Grundlinienimpedanz Z_{ED},
die Differenz (Z_{ES}-Zₘᵢₙ) zwischen der maximal erfassten Impedanz Z_{ES} und der minimal erfassten Impedanz Zₘᵢₙ (Impedanzhub während eines Herzzyklusses),
der Quotient ( (Z_{ES}-Zₘᵢₙ)/T_{ES}) aus Impedanzhub (Z_{ES}-Zₘᵢₙ) und der Zeit T_{ES},
der Quotient ((Z_{ES}-Zₘᵢₙ)/ (T_{ES}-Tₘⱼₙ)), gebildet durch den Impedanzhub geteilt (Z_{ES} - Zₘᵢₙ) durch die Differenz (T_{ES}-Tₘᵢₙ) zwischen T_{ES} und Tₘᵢₙ,
die Maxima Z'ₘₐₓ und Z"ₘₐₓ der ersten und der zweiten Ableitung der intrakardialen Impedanz,
die jeweiligen Zeitabstände T'ₘₐₓ und T"ₘₐₓ. beginnend mit einem QRS-Komplex bis zu dem Zeitpunkt, an dem ein Maximum der ersten bzw. der zweiten Ableitung der intrakardialen Impedanz auftritt.
ist, bei dem eine Stimulationsrate anhand eines für einen physiologischen Bedarf eines Patienten charakteristischen Messwertes bestimmt wird.

18. Herzschrittmacher nach einem der Ansprüche 1 bis 17, **dadurch gekennzeichnet, dass** der Herzschrittmacher (22) als Zweikammerschrittmacher mit wenigstens einer ventrikulären und einer atrialen Erfassungseinheit (VS, AS) für dass Erfassen ventrikulärer bzw. atrialer Ereignisse ausgebildet ist.

19. Herzschrittmacher nach einem der Ansprüche 1 bis 18, **dadurch gekennzeichnet, dass** der Herzschrittmacher (22) als ratenadaptiver Herzschrittmacher ausgebildet

20. Herzschrittmacher nach einem der Ansprüche 1 bis 18, **dadurch gekennzeichnet, dass** der Herzschrittmacher (22) als ratenadaptiver Herzschrittmacher ausgebildet ist, bei dem eine Stimulationsrate anhand einer Auswertung der intrakardialen Impedanz derart eingestellt wird, dass Hub der intrakardialen Impedanz maximiert wird.

## Claims

1. A cardiac pacemaker, comprising a stimulation pulse generator (RVP;
LVP) for biventricular stimulation of a heart, said cardiac pacemaker being intended for connection to at least one right-ventricular electrode (16, 18) for the stimulation of a right ventricle of the heart and to at least one left-ventricular electrode (20) for the stimulation of a left ventricle of the heart and being connected to a control unit (30) and being designed to trigger right-ventricular and left-ventricular stimulation pulses with an interventricular delay time which can be set by means of the control unit, wherein the control unit (30) is connected to an impedance detection unit (34) which is to be connected to intracardiac electrodes (16, 18, 20) and is designed to form, from an input signal formed by the impedance detection unit (34) and dependent on the intracardiac impedance, an output signal indicating an optimum biventricular stimulation mode,
**characterised in that** the control unit (30)
is designed to set the interventricular delay time at which the second derivative of the profile of the intracardiac impedance during a cardiac cycle is at its greatest.

2. The cardiac pacemaker according to Claim 1, **characterised in that** the control unit (30) is designed to trigger various biventricular stimulation modes and to evaluate the intracardiac impedance for each stimulation mode.

3. The cardiac pacemaker according to Claim 1 or 2, **characterised in that** the control unit (30) is designed to form, from an input signal formed by the impedance detection unit (34) and dependent on the intracardiac impedance, an output signal indicating an optimum interventricular delay time.

4. The cardiac pacemaker according to one of Claims 1 to 3, **characterised in that** the control unit (30) is designed to form, from an input signal formed by the impedance detection unit (34) and dependent on the intracardiac impedance, an output signal determining the interventricular delay time.

5. The cardiac pacemaker according to one of Claims 1 to 4, **characterised in that** the interventricular delay time can be set between 20 and 40 ms.

6. The cardiac pacemaker according to one of Claims 1 to 5, in which the stimulation pulse generator (RVP; LVP) is to be connected to different ventricular electrodes or to ventricular electrodes that are variable in terms of their position in the heart, **characterised in that** the control unit (30) is designed to evaluate intracardiac impedances for various electrode configurations or electrode positions and to indicate an optimum electrode position or electrode configuration.

7. The cardiac pacemaker according to one of Claims 1 to 6, **characterised in that** the impedance detection unit is designed to detect the impedance by means of a voltage measurement which takes place between two electrodes (16, 20) of different electrode lines (12, 14).

8. The cardiac pacemaker according to one of Claims 1 to 7, **characterised in that** the cardiac pacemaker (22) is designed to produce a current between a pacemaker housing and an intracardiac electrode for impedance measurement.

9. The cardiac pacemaker according to Claim 7 and 8, **characterised in that** the electrodes for the voltage measurement are different from the electrodes for producing the current for the impedance measurement.

10. The cardiac pacemaker according to Claim 8 or 9, **characterised in that** the cardiac pacemaker is designed to produce a current for the impedance measurement that is of a substantially constant current strength between 100 and 500 µA, preferably 200 µA.

11. The cardiac pacemaker according to one of Claims 8 to 10, **characterised in that** the cardiac pacemaker is designed to produce bi-phase current pulses for impedance measurement.

12. The cardiac pacemaker according to Claim 11, **characterised in that** the cardiac pacemaker is designed to produce the bi-phase current pulses at a repetition rate of 100 to 150 Hz, preferably 128 Hz.

13. The cardiac pacemaker according to Claim 11 or 12, **characterised in that** the cardiac pacemaker is designed to produce bi-phase current pulses with a pulse duration between 20 and 40 µs, preferably approximately 30 µs.

14. The cardiac pacemaker according to one of Claims 1 to 13, **characterised in that** the impedance detection unit (34) or the control unit (30) is designed to average the impedance in a time window of 50 to 300 ms duration.

15. The cardiac pacemaker according to Claim 14, **characterised in that** the impedance detection unit (34) or the control unit (30) is designed to start the time window with the detection of a left-ventricular event (contraction).

16. The cardiac pacemaker according to Claim 14, **characterised in that** the impedance detection unit (34) or the control unit (30) is designed to calculate an intracardiac impedance profile.

17. The cardiac pacemaker according to Claim 14, **characterised in that** the impedance detection unit (34) or the control unit (30) is designed to determine one or more of the following parameters of the intracardiac impedance profile:
Z_{ED} as a baseline for the impedance profile,
Z_{ES} as the maximum impedance occurring within a cardiac cycle,
T_{ES} as the interval between QRS complex (start of the contraction of the heart) and the moment at which the maximum intercardiac impedance Z_{ES} occurs,
Zₘᵢₙ as the minimum impedance during a cardiac cycle,
Tₘᵢₙ as the length of time, starting with a QRS complex, until occurrence of the minimum intracardiac impedance Zₘᵢₙ,
the difference (Z_{ES}-Z_{ED}) between the maximum impedance Z_{ES} and the baseline impedance Z_{ED},
the difference (Z_{ES}-Zₘᵢₙ) between the maximum detected impedance Z_{ES} and the minimum detected impedance Zₘᵢₙ (impedance variation during a cardiac cycle),
the quotient ((Z_{ES}-Zₘᵢₙ)/T_{ES}) from impedance variation (Z_{ES}-Zₘᵢₙ) and the time T_{ES},
the quotient ((Z_{ES}-Zₘᵢₙ)/(T_{ES}-Tₘᵢₙ)), formed by the impedance variation (Z_{ES}-Zₘᵢₙ) divided by the difference (T_{ES}-Tₘᵢₙ) between T_{ES} and Tₘᵢₙ,
the maxima Z'ₘₐₓ and Z"ₘₐₓ of the first and second derivative of the intracardiac impedance,
the respective intervals T'ₘₐₓ and T"ₘₐₓ, starting with a QRS complex until the moment at which a maximum of the first and second derivative of the intracardiac impedance occurs.

18. The cardiac pacemaker according to one of Claims 1 to 17, **characterised in that** the cardiac pacemaker (22) is designed as a dual-chamber pacemaker with at least one ventricular and one atrial detection unit (VS, AS) for the detection of ventricular and atrial events respectively.

19. The cardiac pacemaker according to one of Claims 1 to 18, **characterised in that** the cardiac pacemaker (22) is designed as a rate-adaptive cardiac pacemaker in which a stimulation rate is determined on the basis of a measured value which is characteristic of a physiological demand of a patient.

20. The cardiac pacemaker according to one of Claims 1 to 18, **characterised in that** the cardiac pacemaker (22) is designed as a rate-adaptive cardiac pacemaker in which a stimulation rate is set on the basis of an evaluation of the intracardiac impedance, in such a way that the variation of the intracardiac impedance is maximised.

## Revendications

1. Stimulateur cardiaque, comprenant un générateur d'impulsions de stimulation (RVP ; LVP) pour une stimulation biventriculaire d'un coeur, ledit stimulateur cardiaque étant destiné à la connexion d'au moins une électrode de ventricule droit (16, 18) pour la stimulation d'un ventricule droit du coeur et à au moins une électrode de ventricule gauche (20) pour la stimulation d'un ventricule gauche du coeur et étant connecté à une unité de commande (30) et étant conçu pour déclencher des impulsions de stimulation de ventricule droit et de ventricule gauche avec un temps de retard interventriculaire qui peut être réglé au moyen de l'unité de commande, l'unité de commande (30) étant connectée à une unité de détection d'impédance (34) qui doit être connectée aux électrodes intercardiaques (16, 18, 20) et est conçue pour former, à partir d'un signal d'entrée formé par l'unité de détection d'impédance (34) et dépendant de l'impédance intracardiaque, un signal de sortie indiquant un mode de stimulation biventriculaire optimal,
**caractérisé par le fait que** l'unité de commande (30)
est conçue pour régler le temps de retard intraventriculaire auquel la dérivée seconde du profil de l'impédance intracardiaque pendant un cycle cardiaque est à sa valeur la plus haute.

2. Stimulateur cardiaque selon la revendication 1, **caractérisé par le fait que** l'unité de commande (30) est conçue pour déclencher divers modes de stimulation biventriculaire et pour évaluer l'impédance intracardiaque pour chaque mode de stimulation.

3. Stimulateur cardiaque selon l'une des revendications 1 ou 2, **caractérisé par le fait que** l'unité de commande (30) est conçue pour former, à partir d'un signal d'entrée formé par l'unité de détection d'impédance (34) et dépendant de l'impédance intracardiaque, un signal de sortie indiquant un temps de retard interventriculaire optimal.

4. Stimulateur cardiaque selon l'une des revendications 1 à 3, **caractérisé par le fait que** l'unité de commande (30) est conçue pour former, à partir d'un signal d'entrée formé par l'unité de détection d'impédance (34) et dépendant de l'impédance intracardiaque, un signal de sortie déterminant le temps de retard interventriculaire.

5. Stimulateur cardiaque selon l'une des revendications 1 à 4, **caractérisé par le fait que** le temps de retard interventriculaire peut être réglé entre 20 et 40 ms.

6. Stimulateur cardiaque selon l'une des revendications 1 à 5, dans lequel le générateur d'impulsions de stimulation (RVP ; LVP) doit être connecté aux différentes électrodes ventriculaires ou aux électrodes ventriculaires qui sont variables en termes de leur position dans le coeur, **caractérisé par le fait que** l'unité de commande (30) est conçue pour évaluer les impédances intracardiaques pour différentes configurations d'électrode ou positions d'électrode et pour indiquer une position d'électrode optimale ou une configuration d'électrode optimale.

7. Stimulateur cardiaque selon l'une des revendications 1 à 6, **caractérisé par le fait que** l'unité de détection d'impédance est conçue pour détecter l'impédance au moyen d'une mesure de tension qui a lieu entre deux électrodes (16, 20) de différentes lignes d'électrode (12, 14).

8. Stimulateur cardiaque selon l'une des revendications 1 à 7, **caractérisé par le fait que** le stimulateur cardiaque (22) est conçu pour produire un courant entre un boîtier de stimulateur et une électrode intracardiaque pour une mesure d'impédance.

9. Stimulateur cardiaque selon l'une des revendications 7 et 8, **caractérisé en ce que** les électrodes pour la mesure de tension sont différentes des électrodes pour produire le courant pour la mesure d'impédance.

10. Stimulateur cardiaque selon l'une des revendications 8 ou 9, **caractérisé par le fait que** le stimulateur cardiaque est conçu pour produire un courant pour la mesure d'impédance qui est d'une intensité de courant à peu près constante entre 100 et 500 µA, de préférence de 200 µA.

11. Stimulateur cardiaque selon l'une des revendications 8 à 10, **caractérisé par le fait que** le stimulateur cardiaque est conçu pour produire des impulsions de courant biphasé pour la mesure d'impédance.

12. Stimulateur cardiaque selon la revendication 11, **caractérisé par le fait que** le stimulateur cardiaque est conçu pour produire les impulsions de courant biphasé à une fréquence de répétition de 100 à 150 Hz, de préférence de 128 Hz.

13. Stimulateur cardiaque selon l'une des revendications 11 ou 12, **caractérisé par le fait que** le stimulateur cardiaque est conçu pour produire des impulsions de courant biphasé ayant une durée d'impulsion entre 20 et 40 µs, de préférence d'approximativement 30 µs.

14. Stimulateur cardiaque selon l'une des revendications 1 à 13, **caractérisé par le fait que** l'unité de détection d'impédance (34) ou l'unité de commande (30) est conçue pour faire la moyenne de l'impédance dans une fenêtre de temps d'une durée de 50 à 300 ms.

15. Stimulateur cardiaque selon la revendication 14, **caractérisé par le fait que** l'unité de détection d'impédance (34) ou l'unité de commande (30) est conçue pour démarrer la fenêtre de temps avec la détection d'un événement de ventricule gauche (contraction).

16. Stimulateur cardiaque selon la revendication 14, **caractérisé par le fait que** l'unité de détection d'impédance (34) ou l'unité de commande (30) est conçue pour calculer un profil d'impédance intracardiaque.

17. Stimulateur cardiaque selon la revendication 14, **caractérisé par le fait que** l'unité de détection d'impédance (34) ou l'unité de commande (30) est conçue pour déterminer un ou plusieurs des paramètres suivants de profil d'impédance intracardiaque :
Z_{ED} comme ligne de base pour le profil d'impédance,
Z_{ES} comme impédance maximale ayant lieu à l'intérieur d'un cycle cardiaque,
T_{ES} comme intervalle entre le complexe QRS (début de contraction du coeur) et le moment auquel l'impédance intracardiaque maximale Z_{ES} a lieu,
Zₘᵢₙ comme impédance minimale durant un cycle cardiaque,
Tₘᵢₙ comme longueur de temps, partant avec un complexe QRS, jusqu'à l'apparition de l'impédance intracardiaque minimale Zₘᵢₙ,
la différence (Z_{ES} - Z_{ED}) entre l'impédance maximale Z_{ES} et l'impédance de ligne de base Z_{ED},
la différence (Z_{ES} - Zₘᵢₙ) entre l'impédance détectée maximale Z_{ES} et
l'impédance détectée minimale Zₘᵢₙ (variation d'impédance durant un cycle cardiaque),
le quotient ((Z_{ES} - Zₘᵢₙ)/T_{ES}) de la variation d'impédance ((Z_{ES} - Zₘᵢₙ) et du temps T_{ES},
le quotient ((Z_{ES} - Zₘᵢₙ)/(T_{ES} - Tₘᵢₙ)), formé par la variation d'impédance (Z_{ES} - Zₘᵢₙ) divisée par la différence (T_{ES} - Tₘᵢₙ) entre T_{ES} et Tₘᵢₙ,
les maxima Z'ₘₐₓ et Z"ₘₐₓ des dérivées première et seconde de l'impédance intracardiaque,
les intervalles respectifs T'ₘₐₓ et T"ₘₐₓ, partant avec un complexe QRS jusqu'au moment auquel un maximum des dérivées première et seconde de l'impédance intracardiaque a lieu.

18. Stimulateur cardiaque selon l'une des revendications 1 à 17, **caractérisé par le fait que** le stimulateur cardiaque (22) est conçu comme un stimulateur à double chambre avec au moins une unité de détection ventriculaire et une unité de détection auriculaire (VS, AS) pour la détection d'événements respectivement ventriculaires et auriculaires.

19. Stimulateur cardiaque selon l'une des revendications 1 à 18, **caractérisé par le fait que** le stimulateur cardiaque (22) est conçu comme un stimulateur cardiaque à fréquence asservie dans lequel une fréquence de stimulation est déterminée sur la base d'une valeur mesurée qui est caractéristique d'une demande physiologique d'un patient.

20. Stimulateur cardiaque selon l'une des revendications 1 à 18, **caractérisé par le fait que** le stimulateur cardiaque (22) est conçu comme un stimulateur cardiaque à fréquence asservie dans lequel une fréquence de stimulation est réglée sur la base d'une évaluation de l'impédance intracardiaque, d'une manière telle que la variation de l'impédance intracardiaque est rendue maximale.
